# EUROPÄISCHE PATENTSCHRIFT

(11) **EP 2 841 124 B1**
(45) Veröffentlichungstag und Bekanntmachung des Hinweises auf die Patenterteilung: **19.04.2017**
(21) Anmeldenummer: 13723433.2
(22) Anmeldetag: 26.04.2013
(51) Int. Cl.: A61M 1/10, A61B 5/0215, G01L 9/00

(54) **INTRAVASALE ROTATIONSBLUTPUMPE**
INTRAVASCULAR ROTARY BLOOD PUMP
POMPE À SANG INTRAVASCULAIRE ROTATIVE

(30) Priorität: 27.04.2012 DE 102012207053
(43) Veröffentlichungstag der Anmeldung: 04.03.2015
(73) Patentinhaber: Abiomed Europe GmbH, 52074 Aachen (DE)
(72) Erfinder: SIESS, Thorsten, 52074 Aachen (DE)
(74) Vertreter: Klunker . Schmitt-Nilson . Hirsch
(86) Internationale Anmeldenummer: PCT/EP2013/058715
(87) Internationale Veröffentlichungsnummer: WO 2013/160443

(56) Entgegenhaltungen:
- WO-A1-00/37139
- WO-A1-2011/039091
- WO-A2-02/47751
- US-A1- 2001 051 030
- US-A1- 2009 074 367
- US-A1- 2010 241 008

## Beschreibung

Die Erfindung betrifft eine intravasale Rotationsblutpumpe mit ein oder mehreren Drucksensoren zum Messen von Drücken innerhalb des vaskulären Systems des Patienten, die für den Betrieb der Blutpumpe und/ oder für die Beurteilung des Gesundheitszustands des Herzens des Patienten von Bedeutung sind.

In WO 2011/039091 A1 wird im Zusammenhang mit einem Herzunterstützungssystem ein Druckmesskatheter beschrieben, der einen Katheterschlauch und einen Drucksensor zur Messung des Druckes distal von dem Katheterschlauch aufweist. Konkret weist der Druckmesskatheter einen optischen Drucksensor und ein langgestrecktes Rohr aus Metall oder aus einem hochfesten Kunststoff, zum Beispiel PEEK, auf, durch das eine lose verlegte Lichtleitfaser des optischen Drucksensors verläuft. Am vorderen (distalen) Ende des Druckmesskatheters befindet sich ein Sensorkopf, der nach dem Fabri-Perot-Prinzip arbeitet. Der Sensorkopf besitzt eine Kavität, die einerseits durch eine dünne, druckempfindliche Glasmembran abgeschlossen wird und in die andererseits das Ende der Lichtleitfaser hineinragt. Die druckempfindliche Glasmembran verformt sich in Abhängigkeit von der Größe des auf den Sensorkopf einwirkenden Drucks. Durch die Reflexion an der Glasmembran wird das aus der Lichtleitfaser austretende Licht modulierend reflektiert und wieder in die Lichtleitfaser eingespeist. Am proximalen Ende der Lichtleitfaser befindet sich eine Auswerteeinheit mit integrierter CCD-Kamera, die das erhaltene Licht in Form eines Interferenzmusters auswertet. In Abhängigkeit hiervon wird ein druckabhängiges elektrisches Signal erzeugt. Insgesamt handelt es sich somit um einen optoelektronischen Drucksensor.

Der Druckmesskatheter wird im Zusammenhang mit intravasalen Herzunterstützungssystemen, wie beispielsweise einer intraarteriellen Ballonpumpe (IABP) oder einer intravasalen Rotationsblutpumpe, verwendet, indem das betreffende Herzunterstützungssystem zunächst mittels eines Katheterschlauchs an die gewünschte Stelle des vaskulären Systems des Patienten vorgeschoben wird, also beispielsweise in die Aorta oder in eine Herzkammer. Der Druckmesskatheter einschließlich des die Lichtleitfaser umgebenden Rohres ist relativ zu diesem Katheterschlauch in dessen Längsrichtung verschiebbar und wird nachträglich in das Lumen des Katheterschlauchs eingeführt, durch den Katheterschlauch vorgeschoben und tritt aus dessen Ende aus. Wenn der Sensorkopf die vorgesehene Messstelle erreicht hat, wird das Rohr des Druckmesskatheters zurückgezogen, kann aber auch an Ort und Stelle verbleiben. Im Zusammenhang mit einer Rotationsblutpumpe wird vorgeschlagen, den Druckmesskatheter weit über das distale Ende des Katheterschlauchs hinaus und an der Pumpeneinrichtung der Rotationsblutpumpe vorbei zu schieben, so dass er die Aortenklappe passiert und mit seinem Sensorkopf in den linken Ventrikel hineinragt, um so den Ventrikeldruck zu messen.

Grundsätzlich wünschenswert wäre es, wenn der Sensorkopf des optischen Drucksensors bereits mit der Platzierung der Rotationsblutpumpe an Ort und Stelle gebracht würde, wie dies zum Beispiel im Zusammenhang mit Drucksensoren bekannt ist, bei denen der Druck über einen hydrostatischen Druckübertragungsschlauch an eine extrakorporale Druckmesseinrichtung übertragen wird. So ist beispielsweise aus US 2003/0187322 A1 eine Rotationsblutpumpe bekannt, deren Pumpeinrichtung am distalen Ende eine Strömungskanüle besitzt, in die ein hydrostatischer Druckübertragungsschlauch eingebettet ist, welcher sich bis zum distalen Ende der Strömungskanüle erstreckt und dem dortigen Blutdruck ausgesetzt ist.

Allerdings kann ein optischer Drucksensor, der eine Lichtleitfaser umfasst, nicht ohne weiteres entlang der Strömungskanüle verlegt werden. Denn die Strömungskanüle wird auf ihrem Weg bis zur Platzierung im Herzen starken Biegungen unterworfen, die nicht zu vernachlässigende Zug- und Druckspannungen auf eine entlang der Strömungskanäle verlegte Lichtleitfaser ausüben würden. Selbst wenn die Lichtleitfaser innerhalb eines separaten Druckmesslumens frei verlegt würde, könnten die aufgrund von Relativbewegungen zwischen der Lichtleitfaser und dem Druckmesslumen auftretenden Reibungskräfte so groß sein, dass die Lichtleitfaser bricht bzw. reißt. Dies gilt insbesondere für Lichtleitfasern aus Glas. Zwar sind diese Lichtleitfasern in der Regel mit einer dünnen Kunststoffbeschichtung, wie Polyimid (Kapton), überzogen, die einen Bruchschutz darstellen. Dennoch ist die Gefahr des Brechens nicht ausgeschlossen, und es wäre sehr aufwändig, die im Patienten platzierte Blutpumpe insgesamt ersetzen zu müssen, wenn die Lichtleitfaser tatsächlich bricht.

Aufgabe der vorliegenden Erfindung ist es daher, eine Rotationsblutpumpe mit einem an der Strömungskanüle fixierten Sensorkopf eines optischen Drucksensors derart auszugestalten, dass die Gefahr des Brechens der Lichtleitfaser des optischen Drucksensors weitgehend reduziert ist.

Diese Aufgabe wird durch eine intravasale Rotationsblutpumpe mit den Merkmalen des Anspruchs 1 gelöst. In davon abhängigen Ansprüchen sind vorteilhafte Weiterbildungen und Ausgestaltungen der Erfindung angegeben.

Dementsprechend umfasst eine intravasale Rotationsblutpumpe gemäß einer bevorzugten Ausführungsform der Erfindung einen Katheter, eine distal von dem Katheter angeordnete Pumpeinrichtung, die an ihrem distalen Ende eine biegeflexible Strömungskanüle aufweist, durch die hindurch im Betrieb der Blutpumpe Blut von der Pumpeinrichtung entweder angesaugt oder ausgestoßen wird, und mindestens einen Drucksensor mit mindestens einer Lichtleitfaser. Erfindungsgemäß ist dabei die Lichtleitfaser entlang einer "neutralen Faser" der Strömungskanüle verlegt.

Die "neutrale Faser" der Strömungskanüle verläuft in Längsrichtung der Strömungskanüle und zeichnet sich dadurch aus, dass sie nicht oder zumindest geringer, vorzugsweise deutlich geringer, biegeflexibel ist als der Rest der Strömungskanüle, so dass die Strömungskanüle sich gerade nicht oder jedenfalls nicht so leicht entlang der neutralen Faser biegt, wenn die Blutpumpe mit der Strömungskanüle vorweg durch das vaskuläre System des Patienten geführt wird. Per Definition liegt die neutrale Faser bei Biegung der Kanüle genau zwischen den gestauchten und gestreckten Bereichen und erfährt im Idealfall keine Längenänderung.

Dadurch dass die Lichtleitfaser entlang der neutralen Faser der Strömungskanüle verläuft, also entweder mit einer neutralen Faser zusammenfällt oder in derselben "neutralen Biegeebene" wie die neutrale Faser liegt, erfährt die Lichtleitfaser keinerlei Stauchung oder Dehnung, wenn die Rotationsblutpumpe mit der Strömungskanüle voraus durch das vaskuläre System des Patienten geführt wird und sich dabei den unterschiedlichen Krümmungsradien der Gefäße anpasst.

Im einfachsten Fall kann die Lichtleitfaser dazu innen oder außen an der Strömungskanüle entlang einer neutralen Faser der Strömungskanüle mittels eines elastischen Klebers verklebt werden. Sicherheitshalber ist es aber bevorzugt, die Lichtleitfaser innerhalb eines entlang der neutralen Faser verlegten Gleitrohres frei beweglich anzuordnen, um die Lichtleitfaser so zusätzlich gegen Bruchgefährdung aufgrund von Dehnungen oder Stauchungen zu schützen.

Die neutrale Faser der Strömungskanüle kann in verschiedener Weise realisiert sein. So ist es beispielsweise möglich, durch lokale Verstärkung der Strömungskanüle, etwa durch Einbringen oder Aufbringen eines biegesteifen oder zug-druck-steifen Streifens entlang der Strömungskanüle, eine neutrale Faser sozusagen aufzuprägen. Dies bietet sich insbesondere bei Rotationsblutpumpen an, deren Strömungskanüle sich im Normalzustand geradlinig erstreckt. Diese lokale Verstärkung kann beispielsweise durch ein ausreichend starres Lumen oder Gleitrohr, in dem die Lichtleitfaser verlegt ist, erreicht werden.

Häufig ist der Strömungskanüle aber eine Vorkrümmung vorgegeben. Beim Navigieren der Rotationsblutpumpe durch das vaskuläre System des Patienten biegt sich die Strömungskanüle dann so, dass sich diese vorgegebene Krümmung je nach Biegerichtung verstärkt und verringert. Dementsprechend liegt zwischen dem durch die Vorkrümmung definierten inneren Krümmungsradius und äußeren Krümmungsradius der Strömungskanüle eine neutrale Faser, das heißt insgesamt zwei neutrale Fasern, nämlich auf beiden Seiten der Strömungskanüle. Die Vorkrümmung kann wiederum mittels eines biegesteifen Streifens entlang der neutralen Faser oder Fasern vorgegeben oder stabilisiert werden.

Für den Fall, dass trotz der Verlegung der Lichtleitfaser entlang der neutralen Faser der Strömungskanüle die Gefahr einer Streckung oder Stauchung der Lichtleitfaser besteht, ist es - wie ausgeführt - bevorzugt, die Lichtleitfaser in einem separaten Gleitrohr frei beweglich zu verlegen. Dennoch kann es auch in diesem Fall - wie ebenfalls bereits ausgeführt - zu Faserbrüchen kommen, etwa weil die Faser innerhalb des Gleitrohrs aufgrund hoher Reibung exzessiver Dehnung oder Stauchung ausgesetzt ist. Bevorzugt wird daher für die äußere Oberfläche der Lichtleitfaser und die innere Oberfläche des Gleitrohrs eine Gleitmaterialpaarung mit geringem Reibkoeffizienten gewählt. Dies kann eine Metall-Metall-Gleitmaterialpaarung, eine Metall-Kunststoff-Gleitmaterialpaarung oder eine Kunststoff-Kunststoff-Materialpaarung sein, wobei der Kunststoff vorzugsweise Polytetrafluorethylen (PTFE) ist oder zumindest umfasst.

Dazu ist die Lichtleitfaser außenseitig entsprechend mit Polytetrafluorethylen beschichtet. Herkömmliche Lichtleitfasern sind dagegen üblicherweise mit Polyimid (Kapton) beschichtet. Die Lichtleitfaser kann aber auch eine äußere Metallbeschichtung aufweisen, die zum Beispiel aufgedampft sein kann, um mit der inneren Oberfläche des Gleitrohrs eine Metall-Metall-Gleitmaterialpaarung oder Metall-Kunststoff-Gleitmaterialpaarung zu bilden. Denn Metall hat regelmäßig einen niedrigeren Reibkoeffizienten als die üblichen Kunststoffe.

Das Gleitrohr, in dem die Lichtleitfaser frei beweglich verlegt ist, kann ein einfacher Schlauch sein, insbesondere auch ein dehnbarer Schlauch, was aufgrund der Tatsache seiner Verlegung entlang der neutralen Faser der Strömungskanüle möglich ist. Bevorzugt wird allerdings ein metallisches Material für das Gleitrohr, welches gegebenenfalls innenseitig kunststoffbeschichtet ist, vorzugsweise wieder mit Polytetrafluorethylen (PTFE). Ein metallisches Gleitrohr ist druck- und zugstabil und eignet sich daher dazu, der Strömungskanüle eine neutrale Faser aufzuprägen. Bei solchen Rotationsblutpumpen, deren Strömungskanüle bereits durch Vorkrümmung eine neutrale Faser vorgegeben ist, wird diese neutrale Faser durch das metallische Gleitrohr zusätzlich stabilisiert.

Besonders bevorzugt ist es, wenn das Gleitrohr aus einem eine Formgedächtnislegierung umfassenden Material gebildet ist. Der bekannteste Vertreter einer solchen Formgedächtnislegierung ist das sogenannte "Nitinol". Der besondere Nutzen von Formgedächtnislegierungen liegt im vorliegenden Zusammenhang darin, dass Formgedächtnislegierungen ein superelastisches Verhalten zeigen. Die Strömungskanüle kann dadurch auch extremen Krümmungen folgen, ohne dass das Gleitrohr dabei plastisch verformt wird oder der kreisförmige Querschnitt derart verformt wird, dass der Lichtwellenleiter beschädigt würde. Vielmehr biegt sich das Gleitrohr in die ursprüngliche Form zurück. Aufgrund dieser speziellen Materialeigenschaft kann das Lumen sehr klein ausgelegt werden mit nur geringem Spiel für den Lichtleiter. Folglich kann somit auch die Kanüle weiterhin dünnwandig bleiben.

Ein weiterer Vorteil eines Gleitrohrs aus Formgedächtnismetall liegt in der drastisch reduzierten Bruchempfindlichkeit bei mehrfacher Biege- oder Knickbelastung. Während ein "normales" Metallrohr in diesem Fall leichter bricht und damit eine lokale Schwachstelle darstellt, somit die Wahrscheinlichkeit eines nachfolgenden Faserbruchs sogar erhöht, bleibt das Rohr aus Formgedächtnismetall länger biegeelastisch und verhindert somit weiterhin zuverlässig den Faserbruch.

Weiter bevorzugt ist es, wenn das Gleitrohr, in dem die Lichtleitfaser frei beweglich verlegt ist, mit Flüssigkeit gefüllt ist. Dadurch können die Reibkräfte zwischen der Oberfläche der Lichtleitfaser und der Oberfläche des Gleitrohrs weiter reduziert werden.

Als Lichtleitfaser wird vorzugsweise eine Glasfaser verwendet, weil sie leicht zu handhaben und preiswert sind. Insbesondere gibt es besonders dünne Glasfasern, die aufgrund ihres geringen Durchmessers selbst bei sehr engen Biegeradien nicht brechen. Dies ist im Zusammenhang mit der Anbringung der Lichtleitfaser auf der Strömungskanüle von besonderer Bedeutung, denn es kann vorkommen, dass die Strömungskanüle beim Vorschieben durch die Blutgefäße auf ein Hindernis aufläuft, z.B. den Sinus der Aortenklappe, und sich kurzzeitig um 180° faltet.

Darüber hinaus ist ein geringer Durchmesser der Lichtleitfaser auch von Vorteil, um die Querschnittsabmessungen der Blutpumpe möglichst gering zu halten. Dies gilt insbesondere wenn die Lichtleitfaser bzw. das die Lichtleitfaser enthaltende Gleitrohr außen entlang der Strömungskanüle verläuft. Denn je größer der Durchmesser der Lichtleitfaser bzw. der Durchmesser des Gleitrohrs ist, desto größer sind die Querschnittsabmessungen der Blutpumpe, und dies kann beim Platzieren der Blutpumpe und/oder beim Betrieb der Blutpumpe im vaskulären System des Patienten von Nachteil sein. Daher ist es bevorzugt, eine Lichtleitfaser, insbesondere eine eine Glasfaser umfassende Lichtleitfaser, mit einem Durchmesser von 130 µm oder weniger vorzusehen.

Soweit der Sensorkopf des Drucksensors oder, allgemein ausgedrückt, das distale Ende des Drucksensors außen an der Strömungskanüle fixiert wird, also etwa an einem am distalen Ende der Strömungskanüle liegenden Ansaugkorb, wird der Sensorkopf zumindest teilweise in einer Vertiefung aufgenommen, die in der äußeren Oberfläche der Strömungskanüle vorgesehen ist. Dadurch wird der empfindliche Sensorkopf vor einer Kollision mit einem Schleusenventil oder hämostatischen Ventil geschützt, wenn die Blutpumpe ins Gefäßsystem des Patienten eingeführt wird.

Es kann jedoch sein, dass die Wandstärke der Strömungskanüle nicht ausreicht, um eine Vertiefung mit einer Tiefe herzustellen, in der der Sensorkopf vollständig aufgenommen werden kann, so dass das distale Ende des Drucksensors radial über die Peripherie der Strömungskanüle hinausragt. Insbesondere für diese Fälle ist es vorteilhaft, distal vor dem Sensorkopf eine ebenfalls über die Peripherie der Strömungskanüle hinausragende Wulst vorzusehen, damit das hämostatische Ventil oder Schleusenventil beim Einführen der Blutpumpe in das Gefäßsystem des Patienten nicht am distalen Ende des Drucksensors hängenbleibt. Diese Wulst kann alternativ auch so ausgeführt sein, dass sie nicht nur distal vor dem Sensor sondern auch seitlich oder auch proximal vorliegt. Vorteilhafterweise ist diese U- oder O-förmige Wulst so ausgeführt, dass die Sensorkopfkontur vollständig in die Schutzkontur eintaucht, also keine über die Schutzkontur (Wulst) überstehenden Kanten besitzt. Diese Wulst kann beispielsweise eine Klebstoffwulst sein, die gegebenenfalls auch erst nach dem Fixieren des Sensorkopfs in der Vertiefung aufgebracht wird. Die Wulst kann alternativ auch auf die Strömungskanüle aufgeschweißt oder aufgelötet sein.

Nachfolgend wird die Erfindung anhand der begleitenden Zeichnungen beispielhaft erläutert. Darin zeigen:
Figur 1 eine durch die Aorta verlegte Blutpumpe, die sich durch die Aortenklappe bis in den linken Ventrikel erstreckt, mit integriertem Drucksensor,
Figur 2 einen optischen Drucksensor mit Lichtleitfaser,
Figur 3 die Pumpeinrichtung der Blutpumpe aus Figur 1 in größerem Detail,
Figur 4A, 4B den Ausschnitt A aus Figur 3 in Draufsicht und in Seitenansicht,
Figuren 5A, 5B den Ausschnitt B aus Figur 3 in Draufsicht und in Seitenansicht und
Figur 6 einen Querschnitt durch einen Drucksensor mit in einem Gleitrohr geführter Lichtleitfaser.

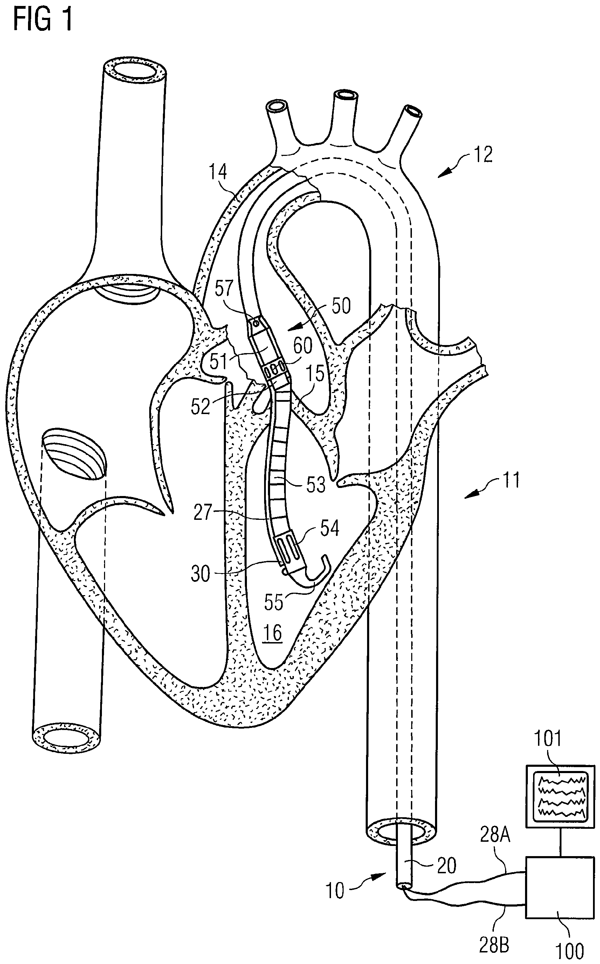

Figur 1 zeigt eine intravasale Blutpumpe mit einem Katheter 10, der retrograd in die Aorta descendens 11 eingeführt ist. Die Aorta descendens ist Bestandteil der Aorta 12, die vom Herzen zunächst aufsteigt und dann abwärts führt und den Aortenbogen 14 aufweist. Am Beginn der Aorta 12 befindet sich die Aortenklappe 15, die den linken Ventrikel 16 mit der Aorta 12 verbindet und durch die hindurch sich die intravasale Blutpumpe erstreckt. Die intravasale Blutpumpe umfasst zusätzlich zu dem Katheter 10 eine am distalen Ende des Katheterschlauchs 20 befestigte rotatorische Pumpeinrichtung 50, die einen Motorteil 51 und einen in axialem Abstand hiervon angeordneten Pumpenteil 52 sowie eine von dem Ansaugende des Pumpenteils 52 in distaler Richtung abstehende Strömungskanüle 53 aufweist, an deren Ende sich ein Saugeinlass 54 befindet. Distal von dem Saugeinlass 54 ist eine weichflexible Spitze 55 vorgesehen, die beispielsweise als "Pigtail" oder in J-Form ausgeführt sein kann. Durch den Katheterschlauch 20 verlaufen verschiedene Leitungen und Einrichtungen, die für den Betrieb der Pumpeinrichtung 50 von Bedeutung sind. Davon sind in Figur 1 lediglich zwei Lichtleitfasern 28A, 28B gezeigt, die mit ihrem proximalen Ende an eine Auswerteeinrichtung 100 angeschlossen sind. Diese Lichtleitfasern 28A, 28B sind jeweils Teil eines optischen Drucksensors, deren Sensorköpfe 30 und 60 sich einerseits außen am Gehäuse des Pumpenteils 52 und andererseits außen in der Nähe des Saugeinlasses 54 befinden. Der von den Sensorköpfen 30 und 60 übermittelte Druck wird in der Auswerteeinrichtung 100 in elektrische Signale umgewandelt und z.B. auf einem Display 101 angezeigt.

Durch die Messung sowohl des Aortendrucks mittels des Sensorkopfs 60 als auch des Ventrikeldrucks mittels des Sensorkopfs 30 sind zusätzlich zum eigentlichen Drucksignal z.B. eine Kontraktibilitätsmessung, bei der die Erholung des Herzens gemessen wird, sowie die Ermittlung der Druckdifferenz, die zur Flussberechnung der Pumpeinrichtung 50 hinzugezogen wird, möglich.

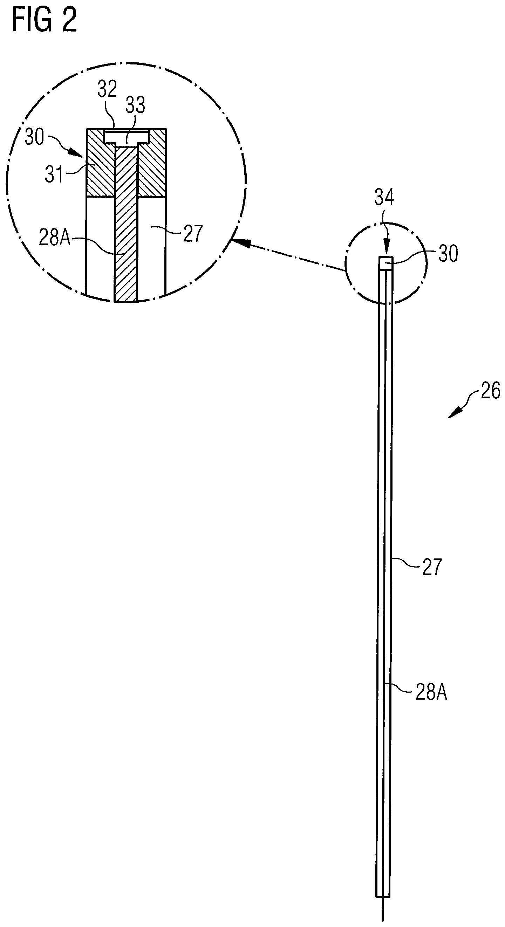

Das Prinzip der elektrooptischen Druckmessung wird nachfolgend anhand der Figur 2 näher erläutert. Figur 2 zeigt einen Druckmesskatheter 26 mit einem Lumen oder Gleitrohr 27, in dem eine Lichtleitfaser 28A (es könnten auch mehrere Lichtleitfasern oder die Lichtleitfaser 28B sein) frei beweglich ist. Das Gleitrohr 27 kann aus einem Polymer, insbesondere Polyurethan, oder vorzugsweise aus Nitinol oder einer anderen Formgedächtnislegierung bestehen, an einer Austrittsstelle 57 aus dem Katheterschlauch 20 austreten (vgl. Figur 1) und an der flexiblen Strömungskanüle 53 z.B. außen entlang verlegt sein. Innerhalb des Katheterschlauchs 20 kann auf das separate Gleitrohr 27 verzichtet werden. Am distalen Ende 34 der Lichtleitfaser 28A weist der Druckmesskatheter einen Sensorkopf 30 mit einem Kopfgehäuse 31 auf, das eine dünne Glasmembran 32 enthält, die eine Kavität 33 abschließt. Die Glasmembran 32 ist druckempfindlich und verformt sich in Abhängigkeit von der Größe eines auf den Sensorkopf 30 einwirkenden Drucks. Durch die Reflexion an der Membran wird das aus der Lichtleitfaser 28A austretende Licht modulierend reflektiert und wieder in die Lichtleitfaser eingekoppelt. Die Einkopplung kann sowohl direkt in die Form 28A erfolgen oder indirekt über einen die Kavität 33 vakuumdicht abschließenden Boden 37. Vorteilhafterweise ist der Boden 37 integraler Bestandteil des Kopfgehäuses 31. Somit kann die Festlegung des Drucks in der Kavität 33 unabhängig von der Montage der Lichtleitfaser 28A erfolgen. Am proximalen Ende der Lichtleitfaser 28A, d. h. in der Auswerteeinrichtung 100, befindet sich eine Digital-Kamera, wie z.B. eine CCD-Kamera oder ein CMOS, die das eintreffende Licht in Form eines Interferenzmusters auswertet. In Abhängigkeit davon wird ein druckabhängiges elektrisches Signal erzeugt. Die Auswertung des von der Kamera gelieferten optischen Bildes bzw. optischen Musters und die Berechnung des Drucks erfolgen durch die Auswerteeinheit 100. Diese gibt die bereits linearisierten Druckwerte an die Steuerung, die auch die Stromzufuhr zu der motorisch betriebenen Pumpeinrichtung 50 in Abhängigkeit von der erfolgten Auswertung des Drucksignals steuert.

Anstelle des in Bezug auf Figur 2 beschriebenen optischen Drucksensors, der nach dem Fabri-Perot-Prinzip arbeitet, können auch andere optische Drucksensoren mit ein oder mehreren Lichtleitfasern verwendet werden.

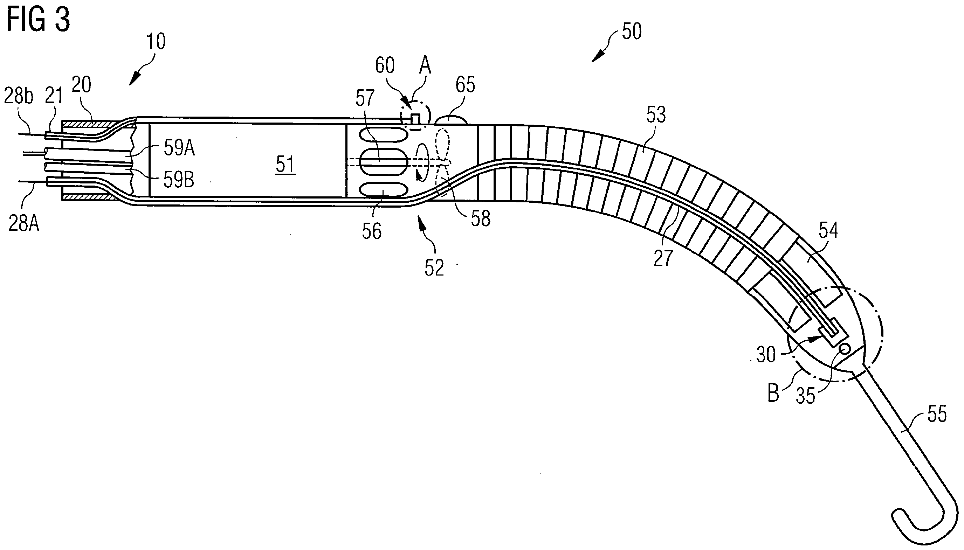

Die Pumpeinrichtung 50 aus Figur 1 ist in Figur 3 in weiterem Detail dargestellt. Zu sehen ist eine vom Motorteil 51 in den Pumpenteil 52 hineinragende Antriebswelle 57, welche ein Flügelrad 58 antreibt, mittels dessen im Betrieb der Blutpumpe Blut durch die Blutdurchtrittsöffnungen 54 am distalen Ende der flexiblen Strömungskanüle 53 hindurch angesaugt und proximal von dem Flügelrad 58 durch die Blutströmungsdurchtrittsöffnungen 56 ausgestoßen wird. Die Pumpeinrichtung 50 kann auch in umgekehrter Richtung fördern, wenn sie entsprechend angepasst wird. Durch den Katheterschlauch 20 des Katheters 10 hindurch zur Pumpeinrichtung 50 führen einerseits die bereits erwähnten Lichtleitfasern 28A, 28B sowie eine Stromversorgungsleitung 59A für den Motorteil 51 und eine Spülflüssigkeitsleitung 59B.

Der Sensorkopf 60 des ersten Drucksensors ist außen am Pumpengehäuse des Pumpenteils 52 fixiert. Die zugehörige Lichtleitfaser 28B ist innerhalb des Katheterschlauchs 20 über eine kurze Distanz von beispielsweise 5 cm in einem dünnen Kunststoffschlauch 21 geführt, um bei starken Krümmungen des Katheters 10 in diesem Bereich des Katheterschlauchs 20 sicherzustellen, dass die Lichtleitfaser 28B nicht bricht. Außerhalb der Pumpeinrichtung 50 ist die Lichtleitfaser 28B frei verlegt und lediglich mittels Klebstoff an der Außenwand der Pumpeinrichtung 50 verklebt. Dadurch werden die äußeren Querschnittsabmessungen der Pumpeinrichtung 50 minimal gehalten. Das Verkleben der Lichtleitfaser 28B ist möglich, weil die Pumpeinrichtung 50 in diesem Bereich starr ist und die Lichtleitfaser 28B sich daher nicht relativ zur Pumpeinrichtung 50 bewegen können muss.

Demgegenüber ist die zum Sensorkopf 30 des zweiten Drucksensors führende Lichtleitfaser 28A entlang der gesamten Peripherie der Pumpeinrichtung 50 in einem Schlauch oder Röhrchen, vorzugsweise einem Nitinol-Röhrchen, frei verlegt, so dass sie sich innerhalb dieses Schlauchs bzw. Röhrchens bei Biegungsänderungen der Strömungskanüle 53 relativ zur Pumpeinrichtung 50 verlagern kann. Der Schlauch bzw. das Röhrchen bilden somit das Gleitrohr 27 für die Lichtleitfaser 28A und verläuft entlang einer neutralen Faser der Strömungskanüle 53 auf der äußeren Oberfläche der Strömungskanüle. Es ist gleichfalls möglich, das Gleitrohr 27 im Inneren der Strömungskanüle 53 zu verlegen, insbesondere wenn ein Druck im Innern der Strömungskanüle 53 gemessen werden soll, oder in die Wandung der Strömungskanüle 53 zu integrieren.

Die neutrale Faser der Strömungskanüle 53 ergibt sich in dem in Figur 3 dargestellten Ausführungsbeispiel dadurch, dass die Strömungskanüle eine Vorkrümmung besitzt, die das Verlegen der Blutpumpe im Gefäßsystem des Patienten erleichtert. Aufgrund dieser Vorkrümmung weist die Strömungskanüle 53 einen inneren Krümmungsradius und einen äußeren Krümmungsradius auf, zwischen denen etwa mittig eine neutrale Biegeebene verläuft. Dort wo diese zentrale Biegeebene die Strömungskanüle 53 schneidet, besitzt die Strömungskanüle neutrale Fasern. Die durch die Vorkrümmung vorgegebene neutrale Faser oder neutrale Ebene der Strömungskanüle 53 wird durch das dort verlegte Gleitrohr 27 verstärkt. Als neutrale Faser der Strömungskanüle im Sinne der vorliegenden Erfindung sind aber auch alle anderen innerhalb der neutralen Ebene verlaufenden Linien zu verstehen, da keine innerhalb dieser Ebene verlaufende Linie oder "Faser" Druck- oder Zugbelastungen ausgesetzt ist, wenn die Vorkrümmung der Strömungskanüle 53 bei ihrem Weg durch das Gefäßsystem des Patienten verstärkt oder verringert wird. Die Verstärkung der neutralen Faser durch das Gleitrohr 27 kann zudem von besonderem Vorteil sein, da hierdurch eine Biegung der Kanüle senkrecht zur Blattebene verhindert oder zumindest verringert wird. Diese Anisotropie kann es dem Anwender einfacher machen, die Kanüle durch das gekrümmte Gefäßsystem vorzuschieben.

Wie eingangs erläutert, kann die Lichtleitfaser 28A auch ohne ein zusätzliches Gleitrohr 27 unmittelbar entlang einer neutralen Faser der Strömungskanüle 53 verlegt und fixiert werden. Das freie Verlegen der Lichtleitfaser 28A innerhalb des entlang der neutralen Faser verlegten Gleitrohrs 27 dient lediglich der zusätzlichen Sicherheit gegen Bruch der Lichtleitfaser 28A.

Der Schlauch und/ oder das Röhrchen des Gleitrohrs 27, in denen die Lichtleitfasern 28A, 28B verlegt sind, können sich bis kurz in den Katheterschlauch 20 hinein erstrecken, können sich aber auch durch den Katheterschlauch 20 vollständig hindurch erstrecken und in einem entsprechenden Stecker am Ende der Leitung zum Einstecken des betreffenden Drucksensors in einen Anschluss der Auswerteeinrichtung 100 enden.

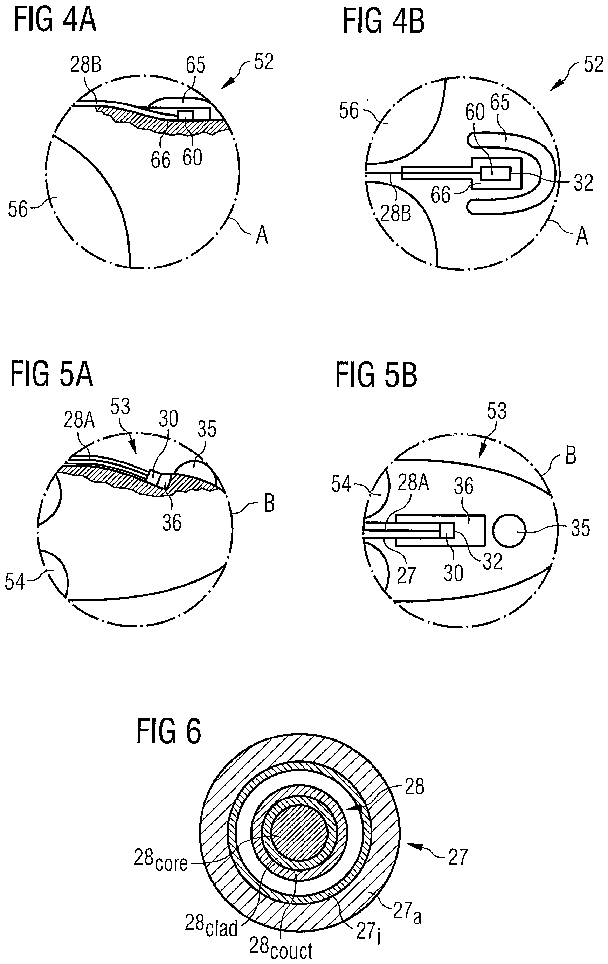

Distal vor und/oder neben und/oder hinter den Sensorköpfen 30 und 60 ist jeweils eine Wulst 35 bzw. 65 vorgesehen, welche die Sensorköpfe 30 und 60 beim Einführen der Blutpumpe durch ein hämostatisches Ventil oder Schleusenventil hindurch vor Beschädigung schützen. Darüber hinaus sind die Sensorköpfe 30 und 60 jeweils in eine Vertiefung 36 bzw. 66 der Pumpeinrichtung 50 eingelassen. Letzteres ist in Figur 3 nicht dargestellt und wird nachfolgend in Bezug auf die Figuren 4A, 4B und 5A, 5B erläutert.

Figur 4A zeigt den Ausschnitt A aus Figur 3 in größerem Detail und teilweise im Querschnitt. Figur 4B zeigt im Grunde den gleichen Ausschnitt A jedoch in Aufsicht von oben. Demnach ist der Sensorkopf 60 in einer auf der äußeren Oberfläche des Pumpenteils 52 vorgesehenen Vertiefung 66 versenkt aufgenommen, wobei die Vertiefung 66 von einer Hufeisen- oder U-förmigen Wulst 65 umgeben ist. Die Wulst könnte auch zu einer O-Form geschlossen sein. Sie ist aufgeklebt oder aufgeschweißt, kann aber auch einen integralen Bestandteil des Pumpenteils 52 bilden. Die Lichtleitfaser 28B ist auf der Oberfläche verklebt und verläuft entlang eines Stegs zwischen zwei Blutströmungsdurchtrittsöffnungen 56.

In ähnlicher Weise (Figuren 5A und 5B) ist auch der Sensorkopf 30 des zweiten Drucksensors in einer Vertiefung 36 auf der äußeren Oberfläche am distalen Ende der Strömungskanüle 53 versenkt aufgenommen. Auch hier verläuft das Nitinol-Röhrchen 27 mit der darin verlegten Lichtleitfaser 28A über einen Steg zwischen zwei Blutströmungsdurchtrittsöffnungen 54 hindurch. Eine punktförmige Wulst 35 distal unmittelbar vor der Vertiefung 36 schützt den Sensorkopf 30 beim Einführen der Blutpumpe vor Kollisionsbeschädigung. Auch die Wulst 35 kann alternativ U-förmig oder O-förmig ausgebildet und insbesondere aufgeklebt, aufgeschweißt oder ein integraler Bestandteil der Strömungskanüle 53 sein.

Der Sensorkopf 30 kann alternativ zusammen mit dem Gleitrohr 27 bis an eine beliebige Stelle der weichflexiblen Spitze 55 reichen und dort z.B. durch die Bewandung der weichflexiblen Spitze 55 mechanisch geschützt sein. Biegungsinduzierte Druckartefakte sind gering, da die Sensormembran orthogonal zur Bewandung angeordnet ist. Lediglich die Klebeverbindung zwischen dem Lichtwellenleiter 34 und dem Sensorkopf muss gegen Verbiegung geschützt werden. Dies kann durch das Röhrchen 27 oder eine zusätzliche Versteifung im Bereich der Klebung erfolgen.

Die Lichtleitfaser 28B wie auch die Lichtleitfaser 28A sind vorzugsweise Glasfasern, die üblicherweise polymerbeschichtet sind. Lichtleitfasern aus Kunststoff sind aber ebenso einsetzbar. Jedoch können Lichtleitfasern aus Glas besonders dünn hergestellt werden, was - insbesondere in Kombination mit dem zusätzlichen Gleitrohr 27 - günstig ist, um den Gesamtquerschnitt der Pumpeinrichtung 50 gering zu halten. Insofern ist es vorteilhaft, Lichtleitfasern mit einem Glaskern zu verwenden, die einen Gesamtdurchmesser von 130 µm nicht überschreiten. Zwar sind derart dünne Lichtleitfasern besonders bruchgefährdet, wenn sie mit Zug- oder Druckkräften beaufschlagt werden. Durch die Anordnung der Lichtleitfaser entlang der neutralen Faser der Strömungskanüle 53 wird dieses Risiko jedoch weitgehend reduziert. Das Gleitrohr 27 kann dann einen Innendurchmesser von nur 150 µm besitzen. Der Außendurchmesser liegt dann geringfügig darüber, beispielsweise bei 220 µm, so dass sich der Gesamtquerschnitt der Pumpeinrichtung 50 nicht nennenswert vergrößert. Denn zu berücksichtigen ist, dass das Gleitrohr 27 zumindest im Bereich des Pumpenteils 52 immer außerhalb der Pumpeinrichtung 50 verlegt werden muss. Röhrchen aus der Formgedächtnislegierung Nitinol sind mit den vorgenannten Innen- und Außendurchmessern kommerziell erhältlich. Es ist aber auch möglich, Gleitrohre, insbesondere Nitinol-Röhrchen, mit größeren Durchmessern zu verwenden, beispielsweise einem Innendurchmesser von 230 µm und einem Außendurchmesser von 330 µm. Die Lichtleitfaser 28A kann dann auch einen entsprechend größeren Durchmesser aufweisen.

Figur 6 zeigt einen Querschnitt durch ein Gleitrohr 27 mit darin verlegter Lichtleitfaser 28, die einen Glasfaserkern 28_{core}, ein sogenanntes ebenfalls aus Glas bestehendes Cladding 28_{clad} und eine äußere Polymerbeschichtung 28_{coat} umfasst. Die unterschiedlichen Brechungsindizes des Lichtleitfaserkerns 28_{core} und des Lichtleitfaser-Claddings 28_{clad} sorgen dafür, dass in den Lichtleitfaserkern eingekoppeltes Licht quasi verlustfrei entlang der Lichtleitfaser 28 geleitet wird. Die äußere Beschichtung 28_{coat} dient zum Schutz der Lichtleitfaser 28 gegen Brechen. Während die Beschichtung 28_{coat} üblicherweise eine Polyimidbeschichtung ist, ist es im Rahmen der vorliegenden Erfindung vorteilhaft, eine möglichst reibungsarme Beschichtung 28_{coat} vorzusehen, insbesondere aus Polytetrafluorethylen (PTFE). Die äußere Beschichtung 28_{coat} kann auch eine metallische Beschichtung sein. Der Glasfaserkern 28_{core} kann einen Durchmesser von z.B. 62,5 µm besitzen. Die Reflexionsschicht könnte dann den Durchmesser auf 80 µm vergrößern. Die Beschichtung 28_{coat} kann eine Dicke von 10 µm besitzen. Der Durchmesser der Lichtleitfaser 28 beträgt vorzugsweise insgesamt ca. 100 µm oder weniger.

Man unterscheidet zwischen normalen Lichtleitfasern und Gradientenindexfasern. Bei Gradientenindexfasern wird das Glasfaser-Cladding 28_{clad} durch viele übereinander liegende Glasschichten mit unterschiedlichen Brechungsindizes gebildet. Die Verwendung von Gradientenindexfasern wird im Zusammenhang mit der vorliegenden Erfindung bevorzugt, weil sie stärker biegbar und verlustfreier sind als einfache Glasfasern.

Das Gleitrohr 27 besteht seinerseits aus einem äußeren Mantel 27a, mit einer innenseitigen Beschichtung 27i. Der äußere Mantel 27a definiert im wesentlichen die Biege- und Dehnungseigenschaften des Gleitrohrs 27, während die innen liegende Beschichtung 27i für die Reduzierung der zwischen dem Gleitrohr 27 und der Lichtleitfaser 28 wirkenden Reibkräfte wesentlich ist. Die innen liegende Beschichtung 27i ist daher vorzugsweise eine metallische Beschichtung oder wiederum eine reibungsarme Polymerbeschichtung, insbesondere aus Polytetrafluorethylen.

Das Gleitrohr 27 kann zusätzlich mit einer Flüssigkeit gefüllt sein, um auftretende Reibkräfte gering zu halten.

## Patentansprüche

1. Intravasale Rotationsblutpumpe, umfassend einen Katheter (10), eine distal von dem Katheter (10) angeordnete Pumpeinrichtung (50), die an ihrem distalen Ende eine biegeflexible Strömungskanüle (53) aufweist, durch die hindurch im Betrieb der Blutpumpe Blut von der Pumpeinrichtung (50) entweder angesaugt oder ausgestoßen wird, und mindestens einen Drucksensor (27, 28A, 30) mit mindestens einer Lichtleitfaser (28A), **dadurch gekennzeichnet, dass** die Lichtleitfaser (28A) entlang einer neutralen Faser der Strömungskanüle (53) verlegt ist.

2. Blutpumpe nach Anspruch 1, wobei die neutrale Faser durch eine Vorkrümmung der Strömungskanüle (53) vorgegeben ist und dementsprechend zwischen einem durch die Vorkrümmung definierten inneren Krümmungsradius und äußeren Krümmungsradius der Strömungskanüle liegt.

3. Blutpumpe nach Anspruch 1 oder 2, wobei die Lichtleitfaser (28A) entlang der neutralen Faser verklebt ist.

4. Blutpumpe nach Anspruch 1 oder 2, wobei die Lichtleitfaser (28A) entlang der neutralen Faser in einem Gleitrohr (27) frei beweglich verlegt ist.

5. Blutpumpe nach Anspruch 4, wobei das Gleitrohr (27) außen entlang der Strömungskanüle (53) verläuft.

6. Blutpumpe nach Anspruch 4 oder 5, wobei eine äußere Oberfläche der Lichtleitfaser (28A) und eine innere Oberfläche des Gleitrohrs (27) eine Gleitmaterialpaarung aus Metall-Metall, Metall-Kunststoff oder Kunststoff-Kunststoff bilden, wobei der Kunststoff vorzugsweise Polytetrafluorethylen (PTFE) umfasst.

7. Blutpumpe nach einem der Ansprüche 4 bis 6, wobei das Gleitrohr (27) aus einem eine Formgedächtnislegierung umfassenden Material gebildet ist.

8. Blutpumpe nach einem der Ansprüche 4 bis 7, wobei das Gleitrohr (27) innenseitig kunststoffbeschichtet ist.

9. Blutpumpe nach einem der Ansprüche 4 bis 8, wobei die Lichtleitfaser (28A) eine äußere Metallbeschichtung (28_{coat}) aufweist.

10. Blutpumpe nach einem der Ansprüche 4 bis 8, wobei die Lichtleitfaser (28A) eine äußere Kunststoffbeschichtung (28_{coat}) aus Polytetrafluorethylen (PTFE) aufweist.

11. Blutpumpe nach einem der Ansprüche 4 bis 10, wobei das Gleitrohr (27) mit Flüssigkeit gefüllt ist.

12. Blutpumpe nach einem der Ansprüche 1 bis 11, wobei die Lichtleitfaser (28A) eine Glasfaser (28_{core}, 28_{clad}, 28_{coat}) umfasst.

13. Blutpumpe nach einem der Ansprüche 1 bis 12, wobei die Lichtleitfaser (28A) einen Durchmesser von 130 µm oder weniger besitzt.

14. Blutpumpe nach einem der Ansprüche 1 bis 13, wobei die Strömungskanüle (53) eine äußere Oberfläche aufweist, in der eine Vertiefung (36) ausgebildet ist, in welcher ein distales Ende (30) des Drucksensors zumindest teilweise angeordnet ist.

15. Blutpumpe nach einem der Ansprüche 1 bis 14, wobei die biegeflexible Strömungskanüle (53) eine weichflexible Spitze (55) aufweist und wobei ein distales Ende (30) des Drucksensors zumindest teilweise in der weichflexiblen Spitze (55) angeordnet ist.

16. Blutpumpe nach einem der Ansprüche 1 bis 15, wobei ein distales Ende (30) des Drucksensors über die Peripherie der Strömungskanüle (53) radial hinausragt, und wobei an der Strömungskanüle (53) distal vor dem distalen Ende (30) des Drucksensors eine ebenfalls über die Peripherie der Strömungskanüle (53) hinausragende Wulst (35) vorgesehen ist.

## Claims

1. An intravascular rotary blood pump, comprising a catheter (10), a pumping device (50) disposed distally of the catheter (10) and having at its distal end a flexurally flexible flow cannula (53) through which blood is either sucked or discharged by the pumping device (50) during operation of the blood pump, and at least one pressure sensor (27, 28A, 30) having at least one optical fiber (28A), **characterized in that** the optical fiber (28A) is laid along a neutral fiber of the flow cannula (53).

2. The blood pump according to claim 1, wherein the neutral fiber is preset by a precurvature of the flow cannula (53) and accordingly lies between an inner radius of curvature and outer radius of curvature of the flow cannula as defined by the precurvature.

3. The blood pump according to claim 1 or 2, wherein the optical fiber (28A) is bonded along the neutral fiber.

4. The blood pump according to claim 1 or 2, wherein the optical fiber (28A) is laid freely movably in a sliding tube (27) along the neutral fiber.

5. The blood pump according to claim 4, wherein the sliding tube (27) extends along the flow cannula (53) on the outside.

6. The blood pump according to claim 4 or 5, wherein an outer surface of the optical fiber (28A) and an inner surface of the sliding tube (27) form a sliding material pairing of metal-metal, metal-plastic or plastic-plastic, the plastic preferably comprising polytetrafluoroethylene (PTFE).

7. The blood pump according to any of claims 4 to 6, wherein the sliding tube (27) is formed from a material comprising a shape-memory alloy.

8. The blood pump according to any of claims 4 to 7, wherein the sliding tube (27) is plastic-coated on the inside.

9. The blood pump according to any of claims 4 to 8, wherein the optical fiber (28A) has an outer metal coating (28_{coat}).

10. The blood pump according to any of claims 4 to 8, wherein the optical fiber (28A) has an outer plastic coating (28_{coat}) made of polytetrafluoroethylene (PTFE).

11. The blood pump according to any of claims 4 to 10, wherein the sliding tube (27) is filled with liquid.

12. The blood pump according to any of claims 1 to 11, wherein the optical fiber (28A) comprises a glass fiber (28_{core}, 28_{clad}, 28_{coat}).

13. The blood pump according to any of claims 1 to 12, wherein the optical fiber (28A) possesses a diameter of 130 µm or less.

14. The blood pump according to any of claims 1 to 13, wherein the flow cannula (53) has an outer surface in which there is configured a depression (36) in which a distal end (30) of the pressure sensor is at least partly disposed.

15. The blood pump according to any of claims 1 to 14, wherein the flexurally flexible flow cannula (53) has a soft-flexible tip (55) and wherein a distal end (30) of the pressure sensor is at least partly disposed in the soft-flexible tip (55).

16. The blood pump according to any of claims 1 to 15, wherein a distal end (30) of the pressure sensor projects radially beyond the periphery of the flow cannula (53), and wherein there is provided on the flow cannula (53) distally before the distal end (30) of the pressure sensor a bulge (35) likewise projecting beyond the periphery of the flow cannula (53).

## Revendications

1. Pompe rotative intravasculaire comprenant un cathéter (10), un dispositif de pompage (50) agencé de manière distale par rapport au cathéter (10) et doté, à son extrémité distale, d'une canule d'écoulement (53) souple en flexion à travers laquelle, lors du fonctionnement de la pompe à sang, du sang est, par le dispositif de pompage (50), soit aspiré, soit expulsé, et au moins un capteur de pression (27, 28A, 30) ayant au moins une fibre conductrice de lumière (28A), **caractérisée en ce que** la fibre conductrice de lumière (28A) est placée le long d'une fibre neutre de la canule d'écoulement (53).

2. Pompe à sang selon la revendication 1, la fibre neutre étant prédéterminée par une précourbure de la canule d'écoulement (53) et étant donc située entre un rayon de courbure interne défini par la précourbure et un rayon de courbure externe de la canule d'écoulement.

3. Pompe à sang selon la revendication 1 ou 2, la fibre conductrice de lumière (28A) étant collée le long de la fibre neutre.

4. Pompe à sang selon la revendication 1 ou 2, la fibre conductrice de lumière (28A) étant placée de manière librement mobile le long de la fibre neutre dans un tube de guidage (27).

5. Pompe à sang selon la revendication 4, le tube de guidage (27) évoluant du côté externe le long de la canule d'écoulement (53).

6. Pompe à sang selon la revendication 4 ou 5, une surface externe de la fibre conductrice de lumière (28A) et une surface interne du tube de guidage (27) constituant un appariement de matériaux de glissement métal-métal, métal-plastique ou plastique-plastique, le plastique comprenant de préférence du polytétrafluoréthylène (PTFE).

7. Pompe à sang selon une des revendications de 4 à 6, le tube de guidage (27) étant constitué par un matériau comprenant un alliage à mémoire de forme.

8. Pompe à sang selon une des revendications de 4 à 7, le tube de guidage (27) étant, en sa face interne, revêtu de plastique.

9. Pompe à sang selon une des revendications de 4 à 8, la fibre conductrice de lumière (28A) comportant un revêtement externe métallique (28_{coat}).

10. Pompe à sang selon une des revendications de 4 à 8, la fibre conductrice de lumière (28A) comportant un revêtement externe en plastique (28_{coat}) en polytétrafluoréthylène (PTFE).

11. Pompe à sang selon une des revendications de 4 à 10, le tube de guidage (27) étant rempli de liquide.

12. Pompe à sang selon une des revendications de 1 à 11, la fibre conductrice de lumière (28A) comprenant une fibre de verre (28_{core}, 28_{clad}, 28_{coat}).

13. Pompe à sang selon une des revendications de 1 à 12, la fibre conductrice de lumière (28A) ayant un diamètre de 130 µm ou moins.

14. Pompe à sang selon une des revendications de 1 à 13, la canule d'écoulement (53) comportant une surface externe dans laquelle un renfoncement (36) est pratiqué, dans lequel une extrémité distale (30) du capteur de pression est au moins partiellement agencée.

15. Pompe à sang selon une des revendications de 1 à 14, la canule d'écoulement (53) souple en flexion comportant une pointe souple en consistance (55), et une extrémité distale (30) du capteur de pression étant au moins partiellement agencée dans la pointe souple en consistance (55).

16. Pompe à sang selon une des revendications de 1 à 15, cependant qu'une extrémité distale (30) du capteur de pression dépasse radialement de la périphérie de la canule d'écoulement (53), et que, à la canule d'écoulement (53), distalement avant l'extrémité distale (30) du capteur de pression, un cordon (35) dépassant aussi de la périphérie de la canule d'écoulement (53) est prévu.
